# FASCICULE DE BREVET EUROPEEN

(11) **EP 3 749 846 B1**
(45) Date de publication et mention de la délivrance du brevet: **16.10.2024**
(21) Numéro de dépôt: 19705129.5
(22) Date de dépôt: 05.02.2019
(51) Int. Cl.: F02M 25/025, F02M 25/022, C02F 1/50, A61L 2/238

(54) **SYSTÈME D'INJECTION DE SOLUTION AQUEUSE POUR VÉHICULE**
EINSPRITZSYSTEM WÄSSERIGER LÖSUNG FÜR FAHRZEUG
AQUEOUS SOLUTION INJECTION SYSTEM FOR VEHICLE

(30) Priorité: 05.02.2018 FR 1850932
(43) Date de publication de la demande: 16.12.2020
(73) Titulaire: Plastic Omnium Advanced Innovation And Research, 1130 Bruxelles (BE)
(72) Inventeur: OP DE BEECK, Joel, 2547 LINT (BE); DUEZ, Laurent, 1180 UCCLE (BE); MONGE-BONINI, Beatriz, 1150 Woluwé Saint-Pierre (BE)
(74) Mandataire: LLR
(86) Numéro de dépôt international: PCT/EP2019/052754
(87) Numéro de publication internationale: WO 2019/149956

(56) Documents cités:
- EP-A2- 2 419 611
- WO-A1-2013/075832
- WO-A1-2016/177556
- US-A1- 2005 155 939
- US-A1- 2008 110 812
- US-A1- 2013 327 419

## Description

L'invention concerne les systèmes d'injection de solution aqueuse pour véhicule.

Les systèmes d'injection d'eau pour véhicule comprennent généralement un réservoir d'eau, une pompe et une ligne d'injection menant à un éjecteur depuis lequel l'eau est éjectée. Après que le système d'injection d'eau a éjecté de l'eau via l'éjecteur, l'eau restant dans la ligne d'injection est purgée et retourne au réservoir. Cependant, au moins une partie de cette eau a été en contact avec de l'air qui est entrée dans la ligne d'injection après l'éjection. Cet air est notamment susceptible de contenir des composés organiques ou des micro-organismes tels que des bactéries. Il existe un risque que les micro-organismes ainsi introduits prolifèrent, ce qui n'est pas souhaitable. En effet, certains micro-organismes tels que des bactéries, des champignons ou des algues sont connus pour être capable d'adhérer à une surface et, à partir de celle-ci, proliférer et sécréter une matrice adhésive et protectrice formant ainsi un biofilm. La prolifération des micro-organismes et la formation d'un biofilm sont susceptibles de réduire au moins partiellement l'espace intérieur d'une conduite. Cette réduction peut être la cause de dysfonctionnements du système en influant notamment sur la pression et le débit avec lequel un liquide s'écoule dans la conduite. La prolifération des micro-organismes présents dans le biofilm peut être telle que le biofilm peut finir par obstruer complètement la conduite de manière à rendre le système inopérant et entraîner un risque important de détérioration des éléments le composant.

Le document EP 2 419 611 décrit un système d'injection de solution aqueuse pour véhicule conforme au préambule de la revendication 1.

Par ailleurs, lorsque la température est très basse, par exemple en hiver, il est important d'éviter que l'eau ne gèle afin de réduire le risque d'endommagement du dispositif. Une manière d'éviter que l'eau ne gèle consiste à chauffer au moins une partie du dispositif. On est alors confronté à deux problèmes, à savoir faire des économies d'énergie et chauffer des parties du dispositif où il n'est pas aisé d'intégrer un système de chauffage.

Un but de l'invention est donc d'améliorer les systèmes d'injection de solution aqueuse pour véhicule.

A cet effet, on prévoit selon l'invention un système d'injection de solution aqueuse de véhicule, comprenant :
- un réservoir de solution aqueuse en matière plastique,
- une pompe d'injection,
- un éjecteur de solution aqueuse,
- une ligne d'injection située entre la pompe et l'éjecteur,
- une ligne de purge située entre la ligne d'injection et le réservoir de solution aqueuse, et
- un raccord de sortie situé entre la ligne d'injection et la ligne de purge, le raccord présentant une face externe et une face interne, caractérisé en ce que le raccord forme un dispositif anti-obstruction apte à limiter ou empêcher la formation d'un biofilm, au moins une portion de la face interne du raccord étant faite ou recouverte d'un métal ou d'un matériau comprenant un métal, le métal étant sélectionné parmi le cuivre, l'argent, le zinc, l'aluminium, le nickel, l'or, le baryum, le plomb, l'étain, le bore, le thallium, l'antimoine, le cobalt, le zirconium, le molybdène, le titane, le fer, le chrome, et un alliage d'au moins deux d'entre eux, dans lequel un manchon réalisé dans le métal ou dans le matériau comprenant le métal est inséré dans le raccord et forme la portion ou toute la face interne du raccord.

On peut par exemple prévoir que le métal est un alliage de cuivre et d'étain (bronze) ou de cuivre et de zinc (laiton). On peut également prévoir que l'alliage comprend du fer et du chrome (acier inoxydable).

La face interne du raccord peut être recouverte du métal ou du matériau comprenant le métal par tout moyen connu de l'homme du métier. Les métaux sélectionnés présentent tous des propriétés biocides. Ces propriétés biocides sont notamment dues à l'effet oligodynamique. De cette façon, lorsque la solution aqueuse passe dans le raccord et est au contact d'au moins une portion de sa surface interne, les micro-organismes présents dans cette solution aqueuse, par exemple des bactéries, des champignons ou des algues, qui tentent d'adhérer à la face interne du raccord faite ou recouverte du métal ou du matériau comprenant le métal sont détruits et ne peuvent donc pas y adhérer. Cette propriété est notamment intéressante car de la solution aqueuse peut rester présente et stagner dans la ligne d'injection après que l'éjection de solution aqueuse a eu lieu puisque la totalité de la solution aqueuse n'est pas forcément purgée après l'éjection. Or, après l'éjection de la solution aqueuse, de l'air entre en contact avec la solution aqueuse présente dans la ligne d'injection, cet air comprenant généralement des micro-organismes tels que des bactéries, mais également d'autres éléments tels que des molécules organiques. Cette solution aqueuse résiduelle contaminée forme un milieu propice au développement de micro-organismes tels que des bactéries, des algues ou des champignons. En empêchant ces micro-organismes d'adhérer à la surface interne du raccord, la formation d'un biofilm est empêchée et, par conséquent, le risque d'obstruction du raccord, qu'elle soit partielle ou totale, est limité voire évité. Le raccord est un passage clé du système d'injection de solution aqueuse pour plusieurs raisons. Tout d'abord, l'espace interne délimité par celui-ci est généralement un des plus petits du système d'injection et est donc celui qui aura tendance à se boucher le plus facilement et le plus rapidement en cas de formation d'un biofilm. De plus, comme indiqué précédemment, le raccord est situé à un endroit du système où la solution aqueuse peut stagner après que l'éjection et la purge aient eu lieu, cette solution aqueuse stagnante étant propice au développement de micro-organismes. Le système selon l'invention permet d'éviter que plus de 20% d'un volume interne délimité par le raccord soit obstrué par un biofilm. On peut même réduire ce risque à 10% ou 5% du volume interne, voire supprimer totalement ce risque. Ainsi, pour les raisons évoquées ci-dessus, le fait que ce soit le raccord qui présente une face interne faite ou recouverte d'un métal ou d'un matériau comprenant un métal tel que décrit précédemment est particulièrement efficace pour éviter la formation d'un biofilm et donc une obstruction du système.

Par « activité biocide », on comprend qu'au moins 50 % des micro-organismes, notamment des bactéries, mis en contact avec la surface interne du raccord sont détruits, par exemple au moins 60 %, de préférence au moins 70 %, de préférence encore au moins 80 %, voire au moins 90 %. Parmi les micro-organismes sensibles au métal ou au matériau comprenant un métal selon l'invention on trouve notamment des bactéries à Gram négatif telles que *Pseudomonas aeruginosa* ou *Escherichia coli,* ainsi que des bactéries à Gram positif telles que *Staphylococcus aureus.* Des levures telles que *Saccharomyces cerevisiae,* et des algues du genre *Chlorella* ou *Stichococcus,* telle que par exemple *Chlorella protothecoides*, sont également sensibles au métal ou au matériau comprenant un métal selon l'invention. D'autres types de micro-organismes, par exemple des champignons, sont également sensibles au métal ou au matériau comprenant un métal selon l'invention. Les micro-organismes ainsi détruits ne sont plus capables d'adhérer à la surface interne du raccord et ne peuvent donc pas former de biofilm et obstruer un volume interne du raccord.

L'expression « solution aqueuse » comprend notamment de l'eau. Cette eau est déminéralisée ou non. Elle comprend également des micro-organismes tels que des bactéries ou non. Cette expression comprend également des mélanges d'eau et d'éthanol, par exemple des mélanges comprenant de l'eau et entre 10% et 55% en volume d'éthanol, de préférence entre 20% et 40% en volume d'éthanol. De telles proportions d'éthanol évitent que l'eau présente dans la solution aqueuse ne gèle. On peut prévoir que l'expression « solution aqueuse » ne comprend pas des solutions aqueuses comprenant de l'urée, comme par exemple des solutions d'AdBlue^{®}. En effet, la présence de métaux dans des systèmes utilisant des solutions aqueuses d'urée (par exemple des systèmes utilisant la technique de réduction catalytique sélective (SCR)) n'est pas souhaitée car ces dernières ont un effet corrosif sur les métaux.

Par ailleurs, au moins une partie du système d'injection peut être chauffée. Ce chauffage est notamment nécessaire lorsque la température est trop basse et qu'un risque de gel existe. Dans le cas présent, le métal ou le matériau comprenant le métal sont des conducteurs thermiques de sorte que si un élément du dispositif est chauffé, le métal ou le matériau comprenant le métal transfère par conduction la chaleur depuis l'élément qui est chauffé vers le raccord de sortie. De préférence, l'élément qui est chauffé est un élément du dispositif spatialement proche du raccord de sortie. On peut par exemple prévoir que la ligne d'injection ou la ligne de purge est chauffée. L'avantage de cette caractéristique réside aussi dans l'économie d'énergie qui est réalisée puisqu'on réutilise l'énergie déjà utilisée pour chauffer le premier élément afin de chauffer le raccord.

Par l'expression « faite ou recouverte d'un métal » on comprend que la portion de la surface interne est faite ou recouverte d'un matériau fait à 100 % du métal. Par exemple, la portion de la surface interne est faite ou recouverte d'un matériau fait à 100% de cuivre, à 100 % d'argent, à 100 % de zinc, à 100 % d'aluminium, à 100 % de nickel, à 100 % d'or, à 100 % de baryum, à 100 % de plomb, à 100 d'étain, à 100% de bore, à 100 % de thallium, à 100 % d'antimoine, à 100 % de cobalt, à 100 % de zirconium, à 100 % de molybdène, à 100 % de titane, à 100 % de fer, à 100% de chrome ou à 100 % d'un alliage d'au moins deux de ces métaux, par exemple d'un alliage de cuivre et de zinc (laiton) ou d'un alliage de cuivre et d'étain (bronze).

De préférence, le métal est sélectionné parmi le cuivre, l'argent et le zinc.

De manière préférée, toute la face interne du raccord est faite du métal ou du matériau comprenant le métal.

On augmente ainsi la surface sur laquelle les micro-organismes ne peuvent pas adhérer pour former de biofilms et donc on réduit encore le risque d'obstruction, même partielle, d'un volume interne délimité par le raccord.

Selon l'invention, un manchon réalisé dans le métal ou dans le matériau comprenant le métal est inséré dans le raccord et forme la portion ou toute la face interne du raccord.

Le manchon est de préférence de forme circulaire et d'un diamètre légèrement inférieur à celui du diamètre interne du raccord de façon à pouvoir être logé aisément et de façon stable à l'intérieur de celui-ci.

On peut prévoir qu'une paroi du raccord comprend au moins deux matériaux, un de ces matériaux étant le métal ou le matériau comprenant le métal qui recouvre la portion ou toute la face interne du raccord et qui forme une couche d'une épaisseur comprise entre 1 µm et 10 cm.

On peut par exemple prévoir une couche ayant une épaisseur permettant de remplir presque en totalité un diamètre interne du raccord.

De manière avantageuse, le métal étant un premier métal, le raccord comprend un corps fait en un second métal, le corps étant recouvert sur au moins une portion d'une face interne de celui-ci par le premier métal, le premier métal formant au moins une portion de la face interne du raccord.

On peut prévoir que le corps qui est fait dans le second métal forme également une portion de la face interne du raccord. On peut également prévoir que le second métal est choisi parmi le cuivre, l'argent, le zinc, l'aluminium, le nickel, l'or, le baryum, le plomb, l'étain, le bore, le thallium, l'antimoine, le cobalt, le zirconium, le molybdène, le titane, le fer, le chrome, et un alliage d'au moins deux d'entre eux, par exemple un alliage de cuivre et de zinc (laiton) ou un alliage de cuivre et d'étain (bronze). On peut aussi prévoir que les premier et second métaux formant chacun une portion de la face interne du raccord sont deux métaux différents qui présentent des propriétés biocides différentes, de préférence des propriétés biocides complémentaires.

On peut également prévoir que le corps est fait d'un alliage métallique, d'un alliage comprenant un acier, d'un alliage comprenant un acier inoxydable ou d'un alliage comprenant de l'aluminium.

On peut par exemple prévoir que le corps est en acier inoxydable et que le premier métal recouvrant le corps est un alliage de cuivre et d'argent. De préférence, cet alliage de cuivre et d'argent comprend 10 % en poids d'argent.

De manière préférée, le raccord forme un mamelon ou un manchon.

Avantageusement, le matériau comprenant un métal comprend 60 % à 95 % en poids de cuivre, 3 % à 45 % en poids de Zinc, ou 90 % à 99 % en poids d'argent.

De préférence, le matériau comprenant le métal a une conductivité thermique supérieure à 0,5 W.m⁻¹.K⁻¹ à 20°C.

On peut par exemple prévoir que le matériau comprenant un métal a une conductivité thermique supérieure à 20 W.m⁻¹.K⁻¹ à 20°C, de préférence supérieure à 40 W.m⁻¹.K⁻¹ à 20°C, voire supérieure à 60 W.m⁻¹.K⁻¹ à 20°C. Une grande valeur de conductivité thermique permet une meilleure conduction de la chaleur depuis un élément chauffé jusqu'au raccord. Une meilleure conduction de la chaleur permet notamment de faire des économies en termes d'énergie consommée.

De manière avantageuse, le matériau comprenant un métal comprend une matrice polymérique dans laquelle le métal est dispersé sous forme de charge.

Le matériau est de cette façon plus léger et moins coûteux à produire qu'un matériau fait uniquement de métal. On peut par exemple prévoir que la matrice polymérique est du polyéthylène haute densité (HDPE).

Avantageusement, le matériau comprenant un métal comprend un acier inoxydable ou un alliage comprenant un acier inoxydable.

L'acier inoxydable est connu pour avoir une activité antimicrobienne comme cela est par exemple montré dans le document WO1999064640 A1.

Dans un mode de réalisation, le matériau comprenant un métal comprend un acier inoxydable comprenant 2,5 % à 4,5 % en poids de cuivre, 0,04 % à 0,06 % en poids d'argent, ou une quantité égale ou supérieure à 0,0005 % en poids d'un oxyde d'argent.

On peut également prévoir que le matériau comprenant un métal comprend un alliage métallique, un alliage comprenant un acier, ou un alliage comprenant de l'aluminium.

Dans un mode de réalisation préférée, la solution aqueuse est de l'eau.

L'invention concerne également un véhicule comprenant un système d'injection de solution aqueuse tel que décrit précédemment.

On peut prévoir que le système d'injection de solution aqueuse est configuré pour injecter la solution aqueuse sur un composant ou un système du véhicule présent entre le point d'aspiration d'air du moteur et la chambre de combustion (y compris la chambre de combustion elle-même).

On peut également prévoir que le système d'injection de solution aqueuse est configuré pour injecter la solution aqueuse dans le système d'alimentation en carburant, entre le point d'aspiration du réservoir à carburant jusqu'au point d'injection dans le moteur.

On décrit également un procédé de bio-décontamination d'un système d'injection de solution aqueuse de véhicule, dans lequel :
- de la solution aqueuse est pompée depuis un réservoir en matière plastique vers un éjecteur à travers une ligne d'injection,
- la solution aqueuse pompée est éjectée par l'éjecteur,
- après l'éjection, on purge la solution aqueuse restée dans la ligne d'injection, au cours de la purge, la solution aqueuse est mise en contact avec au moins une surface en métal ou en matériau comprenant au moins un métal, le métal étant sélectionné parmi le cuivre, l'argent, le zinc, l'aluminium, le nickel, l'or, le baryum, le plomb, l'étain, le bore, le thallium, l'antimoine, le cobalt, le zirconium, le molybdène, le titane, le fer, le chrome, et un alliage d'au moins deux d'entre eux.

On peut par exemple prévoir que le métal est un alliage de cuivre et d'étain (bronze) ou de cuivre et de zinc (laiton). On peut également prévoir que l'alliage comprend du fer et du chrome (acier inoxydable).

Nous allons maintenant présenter un mode de réalisation de l'invention donné à titre d'exemple non limitatif et à l'appui des figures annexées sur lesquelles :
- la figure 1 est une vue en coupe longitudinale d'un système d'injection de solution aqueuse selon l'invention ;
- la figure 2 est une représentation schématique du système d'injection de solution aqueuse de la figure 1 ; et
- la figure 3 est une vue en coupe longitudinale du système d'injection de solution aqueuse de la figure 1 à différentes étape d'un procédé de bio-décontamination selon l'invention.

Nous allons présenter un mode de réalisation du système d'injection d'eau montrant les avantages de l'invention.

Dans le cas présent, on met en oeuvre l'invention dans un véhicule fonctionnant avec un moteur thermique, par exemple un moteur à essence. Bien entendu, d'autres types de véhicules peuvent être envisagés.

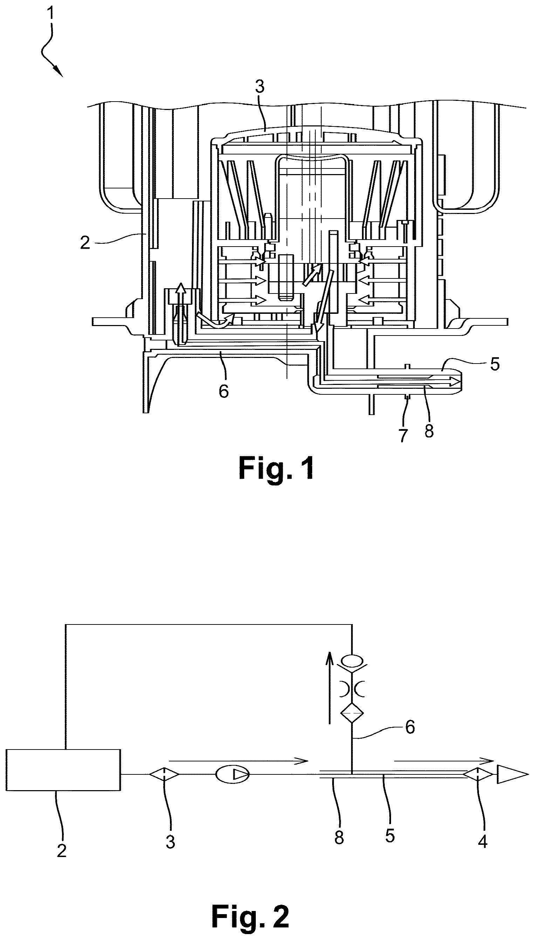

Un système d'injection d'eau 1 selon l'invention comprend un réservoir d'eau 2 en matière plastique, une pompe d'injection 3, un éjecteur 4, une ligne d'injection 5, une ligne de purge 6 et un raccord de sortie 7 (voir figures 1 à 3).

Dans le cas présent, le raccord de sortie 7 est un mamelon. On peut bien entendu prévoir que le raccord de sortie 7 est une autre pièce, par exemple un manchon. Le raccord 7 est disposé entre la pompe d'injection 3 et la ligne d'injection 5. Il est également disposé entre la ligne d'injection 5 et la ligne de purge 6. Le raccord 7 a une forme générale cylindrique circulaire creuse.

Un manchon 8 en métal est présent à l'intérieur du raccord 7. Le manchon 8 a une forme générale cylindrique circulaire creuse. Il a un diamètre externe légèrement inférieur au diamètre interne du raccord 7 de sorte qu'il peut être facilement inséré dans celui-ci de façon stable. Dans le cas présent, le manchon 8 est en cuivre. Bien entendu, on peut prévoir que le manchon est fait d'un autre métal, notamment d'un métal sélectionné parmi l'argent, le zinc, l'aluminium, le nickel, l'or, le baryum, le plomb, l'étain, le bore, le thallium, l'antimoine, le cobalt, le zirconium, le molybdène, le titane, le fer, le chrome, et un alliage d'au moins deux d'entre eux, par exemple un alliage de cuivre et de zinc (laiton) ou d'un alliage de cuivre et d'étain (bronze). Dans un mode de réalisation alternatif, on peut prévoir que le manchon est fait d'une matière comprenant du métal et une autre matière, par exemple une matrice polymérique (par exemple du polyéthylène haute densité ou HDPE) dans laquelle le métal (par exemple le zinc) est dispersé sous forme de charge.

La pompe d'injection 3 est apte à pomper l'eau présente dans le réservoir d'eau et à l'injecter dans la ligne d'injection 5 en passant par le raccord 7.

La ligne d'injection 5 présente deux extrémités, une raccordée au raccord 7 et une raccordée à l'éjecteur 4.

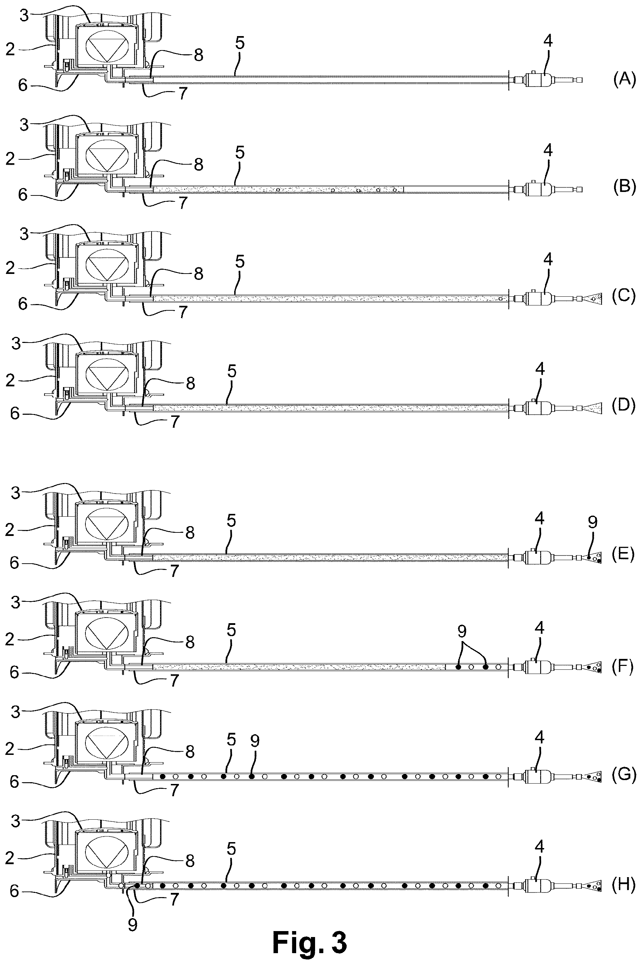

Les différentes étapes du fonctionnement du système d'injection d'eau 1 sont représentées à la figure 3 (étapes A à H).

Dans un premier temps, la pompe 3 et la ligne d'injection 5 sont vides (A).

Ensuite, la pompe 3 est mise en marche et elle, ainsi que la ligne d'injection 5, se remplissent d'eau pompée dans le réservoir 2 (B).

Les gaz qui étaient présents dans la ligne d'injection 5 sortent de celle-ci au niveau de l'éjecteur 4. Une fois que l'eau arrive au niveau de l'éjecteur 4, elle est éjectée hors de la ligne d'injection 5 par celui-ci (C, D).

Une fois que la quantité d'eau désirée a été éjectée, la pompe 3 est arrêtée et l'eau n'est plus éjectée de la ligne d'injection 5 (E).

L'eau restant dans la ligne de d'injection 5 commence alors à être purgée. Lors de la purge, de l'air entre dans la ligne d'injection 5 (F). Cet air comprend des contaminants 9 tels que des bactéries ou des molécules organiques. Au cours de la purge, l'eau est conduite depuis la ligne d'injection 5 vers la ligne de purge 6 en passant par le raccord 7 et donc par le manchon 8. Comme indiqué précédemment, dans le cas présent, ce manchon 8 est en cuivre. Le manchon 8 présente ainsi une activité biocide. A la fin de la purge, il reste de l'eau qui a été contaminée par des contaminants 9 qui stagne dans le raccord 7 et donc dans le manchon 8. Ces contaminants peuvent notamment être des micro-organismes tels que des bactéries, des champignons ou des algues. L'activité biocide du cuivre du manchon 8 empêche les micro-organismes de former un biofilm sur la surface interne 8 du manchon. En effet, l'activité biocide du cuivre du manchon 8 fait que, même si les micro-organismes arrivent à adhérer à la surface interne du manchon 8, ils n'ont pas le temps de proliférer ou de secréter une matrice formant par la suite un biofilm avant d'être détruits par le cuivre. L'obstruction du dispositif est ainsi évitée. Par ailleurs, on peut noter que l'eau qui passe par le raccord 7 et donc par le manchon 8 est ainsi décontaminée au moins en partie des contaminants 9 qui étaient contenus dans l'air et qui se sont répandus et développés dans l'eau (G, H). De cette façon, l'eau qui retourne dans le réservoir 2 ne contient pas ou peu de micro-organismes, par exemple des bactéries, encore capables de se développer et de potentiellement endommager le système.

Il est possible d'utiliser le dispositif selon l'invention pour une solution aqueuse autre que de l'eau, par exemple pour une solution aqueuse comprenant un mélange d'eau et d'éthanol.

## Revendications

1. Système d'injection de solution aqueuse (1) de véhicule, comprenant
- un réservoir de solution aqueuse (2) en matière plastique,
- une pompe d'injection (3),
- un éjecteur de solution aqueuse (4),
- une ligne d'injection (5) située entre la pompe et l'éjecteur,
- une ligne de purge (6) située entre la ligne d'injection et le réservoir de solution aqueuse, et
- un raccord (7) de sortie situé entre la ligne d'injection (5) et la ligne de purge (6), le raccord présentant une face externe et une face interne et formant un dispositif anti-obstruction apte à limiter ou empêcher la formation d'un biofilm, au moins une portion de la face interne du raccord étant faite ou recouverte d'un métal ou d'un matériau comprenant un métal, le métal étant sélectionné parmi le cuivre, l'argent, le zinc, l'aluminium, le nickel, l'or, le baryum, le plomb, l'étain, le bore, le thallium, l'antimoine, le cobalt, le zirconium, le molybdène, le titane, le fer, le chrome, et un alliage d'au moins deux d'entre eux,
le système étant **caractérisé par** un manchon (8) réalisé dans le métal ou dans le matériau comprenant le métal inséré dans le raccord (7) et formant la portion ou toute la face interne du raccord.

2. Système d'injection de solution aqueuse (1) de véhicule selon la revendication 1, dans lequel le métal est sélectionné parmi le cuivre, l'argent et le zinc.

3. Système d'injection de solution aqueuse (1) de véhicule selon l'une quelconque des revendications précédentes, dans lequel toute la face interne du raccord (7) est faite du métal ou du matériau comprenant le métal.

4. Système d'injection de solution aqueuse (1) de véhicule selon l'une quelconque des revendications précédentes, dans lequel le métal étant un premier métal, le raccord (7) comprend un corps fait en un second métal, le corps étant recouvert sur au moins une portion d'une face interne de celui-ci par le premier métal, le premier métal formant au moins une portion de la face interne du raccord.

5. Système d'injection de solution aqueuse (1) de véhicule selon l'une quelconque des revendications précédentes, dans lequel le raccord (7) forme un mamelon ou un manchon.

6. Système d'injection de solution aqueuse (1) de véhicule selon l'une quelconque des revendications précédentes dans lequel le matériau comprenant un métal comprend 60 % à 95 % en poids de cuivre, 3 % à 45 % en poids de Zinc, ou 90 % à 99 % en poids d'argent.

7. Système d'injection de solution aqueuse (1) de véhicule selon l'une quelconque des revendications précédentes, dans lequel le matériau comprenant un métal a une conductivité thermique supérieure à 0,5 W.m⁻¹.K⁻¹ à 20°C.

8. Système d'injection de solution aqueuse (1) de véhicule selon l'une quelconque des revendications précédentes, dans lequel le matériau comprenant un métal comprend une matrice polymérique dans laquelle le métal est dispersé sous forme de charge.

9. Système d'injection de solution aqueuse (1) de véhicule selon l'une quelconque des revendications précédentes, dans lequel le matériau comprenant un métal comprend un acier inoxydable ou un alliage comprenant un acier inoxydable.

10. Système d'injection de solution aqueuse (1) de véhicule selon l'une quelconque des revendications précédentes, dans lequel la solution aqueuse est de l'eau.

11. Véhicule comprenant un système d'injection de solution aqueuse (1) selon l'une quelconque des revendications 1 à 10.

## Patentansprüche

1. Einspritzsystem (1) für eine wässrige Lösung eines Fahrzeugs, aufweisend
- einen Behälter (2) für die wässrige Lösung aus Kunststoff,
- eine Einspritzpumpe (3),
- einen Ejektor (4) für die wässrige Lösung,
- eine Einspritzleitung (5), die sich zwischen der Pumpe und dem Ejektor befindet,
- eine Entlüftungsleitung (6), die sich zwischen der Einspritzleitung und dem Behälter für die wässrige Lösung befindet, und
- einen Auslassverbindungsstück (7), der sich zwischen der Einspritzleitung (5) und der Entlüftungsleitung (6) befindet, wobei das Verbindungsstück eine Außenseite und eine Innenseite aufweist und eine Antiverstopfungsvorrichtung bildet, die geeignet ist, die Bildung eines Biofilms zu begrenzen oder zu verhindern, wobei mindestens ein Teil der Innenseite des Verbindungsstücks aus einem Metall oder einem Material, das ein Metall enthält, hergestellt oder damit bedeckt ist, wobei das Metall ausgewählt ist aus Kupfer, Silber, Zink, Aluminium, Nickel, Gold, Barium, Blei, Zinn, Bor, Thallium, Antimon, Kobalt, Zirkonium, Molybdän, Titan, Eisen, Chrom und einer Legierung aus mindestens zwei dieser Metalle,
wobei das System **gekennzeichnet ist durch** eine Hülse (8), die aus dem Metall oder aus dem das Metall aufweisenden Material hergestellt ist, die in das Verbindungsstück (7) eingesetzt ist und den Abschnitt oder die gesamte Innenseite des Verbindungsstücks bildet.

2. Einspritzsystem (1) für eine wässrige Lösung eines Fahrzeugs nach Anspruch 1, wobei das Metall aus Kupfer, Silber und Zink ausgewählt ist.

3. Einspritzsystem (1) für eine wässrige Lösung eines Fahrzeugs nach einem der vorhergehenden Ansprüche, wobei die gesamte Innenseite des Verbindungsstücks (7) aus dem Metall oder dem das Metall aufweisenden Material hergestellt ist.

4. Einspritzsystem (1) für eine wässrige Lösung eines Fahrzeugs nach einem der vorhergehenden Ansprüche, wobei das Metall ein erstes Metall ist, das Verbindungsstück (7) einen Körper aus einem zweiten Metall aufweist, wobei der Körper auf mindestens einem Teil einer Innenfläche desselben mit dem ersten Metall bedeckt ist, wobei das erste Metall mindestens einen Teil der Innenfläche des Verbindungsstücks bildet.

5. Einspritzsystem (1) für eine wässrige Lösung eines Fahrzeugs nach einem der vorhergehenden Ansprüche, wobei das Verbindungsstück (7) einen Nippel oder eine Muffe bildet.

6. Einspritzsystem (1) für eine wässrige Lösung eines Fahrzeugs nach einem der vorhergehenden Ansprüche, wobei das Material, das ein Metall aufweist, 60 Gew.-% bis 95 Gew.-% Kupfer, 3 Gew.-% bis 45 Gew.-% Zink oder 90 Gew.-% bis 99 Gew.-% Silber aufweist.

7. Einspritzsystem (1) für eine wässrige Lösung eines Fahrzeugs nach einem der vorhergehenden Ansprüche, wobei das Material, das ein Metall aufweist, eine Wärmeleitfähigkeit von mehr als 0,5 W.m⁻¹.K⁻¹ bei 20°C hat.

8. Einspritzsystem (1) für eine wässrige Lösung eines Fahrzeugs nach einem der vorhergehenden Ansprüche, wobei das ein Metall aufweisend Material eine Polymermatrix aufweist, in der das Metall als Füllstoff dispergiert ist.

9. Einspritzsystem (1) für eine wässrige Lösung eines Fahrzeugs nach einem der vorhergehenden Ansprüche, wobei das Material, das ein Metall aufweist, einen rostfreien Stahl oder eine Legierung aufweist, die einen rostfreien Stahl aufweist.

10. Einspritzsystem (1) für eine wässrige Lösung eines Fahrzeugs nach einem der vorhergehenden Ansprüche, wobei die wässrige Lösung Wasser ist.

11. Fahrzeug mit einem Einspritzsystem (1) für eine wässrige Lösung nach einem der Ansprüche 1 bis 10.

## Claims

1. Injection system (1) for an aqueous solution for a vehicle, comprising:
- a tank (2) for an aqueous solution made of plastic,
- an injection pump (3),
- an ejector (4) for an aqueous solution,
- an injection line (5) located between the pump and the ejector,
- a purge line (6) located between the injection line and the tank for aqueous solution, and
- an outlet fitting (7) located between the injection line (5) and the purge line (6), the fitting having an external face and an internal face and forming an anti-clogging device capable of limiting or preventing the formation of a biofilm, at least a portion of the internal face of the fitting being made of or coated with a metal or a material comprising a metal, the metal being selected from copper, silver, zinc, aluminum, nickel, gold, barium, lead, tin, boron, thallium, antimony, cobalt, zirconium, molybdenum, titanium, iron, chromium, and an alloy of at least two of them,
the system being **characterized by** a sleeve (8) made of the metal or material comprising the metal inserted into the fitting (7) and forming the portion or the entire internal face of the fitting.

2. An Injection system (1) for an aqueous solution for a vehicle according to claim 1, wherein the metal is selected from copper, silver and zinc.

3. An Injection system (1) for an aqueous solution for a vehicle according to any one of the preceding claims, wherein the entire internal face of the fitting (7) is made of the metal or the material comprising the metal.

4. An Injection system (1) for an aqueous solution for a vehicle according to any one of the preceding claims, wherein the metal being a first metal, the fitting (7) comprises a body made of a second metal, the body being coated on at least a portion of an internal face thereof by the first metal, the first metal forming at least a portion of the inner face of the fitting.

5. An Injection system (1) for an aqueous solution for a vehicle according to any one of the preceding claims, wherein the fitting (7) forms a nipple or sleeve.

6. An Injection system (1) for an aqueous solution for a vehicle according to any one of the preceding claims, wherein the material comprising a metal comprises 60% to 95% by weight of copper, 3% to 45% by weight of zinc, or 90% to 99% by weight of silver.

7. An Injection system (1) for an aqueous solution for a vehicle according to any one of the preceding claims, wherein the material comprising a metal has a thermal conductivity higher than 0.5 W.m-¹. K⁻¹ at 20°C.

8. An Injection system (1) for an aqueous solution for a vehicle according to any one of the preceding claims, wherein the material comprising a metal comprises a polymeric matrix in which the metal is dispersed as a filler.

9. An Injection system (1) for an aqueous solution for a vehicle according to any one of the preceding claims, wherein the material comprising a metal comprises a stainless steel or an alloy comprising a stainless steel.

10. An Injection system (1) for an aqueous solution for a vehicle according to any one of the preceding claims, wherein the aqueous solution is water.

11. A vehicle comprising an Injection system (1) for an aqueous solution according to any one of claims 1 to 10.
